# EUROPEAN PATENT SPECIFICATION

(11) **EP 3 459 438 B1**
(45) Date of publication and mention of the grant of the patent: **09.12.2020**
(21) Application number: 17193220.5
(22) Date of filing: 26.09.2017
(51) Int. Cl.: A61B 5/00, G06T 7/00

(54) **DEVICE AND METHOD FOR DETERMING DENTAL PLAQUE**
VORRICHTUNG UND VERFAHREN ZUR BESTIMMUNG VON ZAHNBELAG
DISPOSITIF ET PROCÉDÉ DE DÉTERMINATION DE PLAQUE DENTAIRE

(43) Date of publication of application: 27.03.2019
(73) Proprietor: The Procter & Gamble Company, Cincinnati, OH 45202 (US)
(72) Inventor: ENGELMOHR, Reiner, 61476 Kronberg (DE); ADAM, Ralf, 61476 Kronberg (DE); KOCH, Rolf, 8050 Zürich (CH); BAYER, Jeronimo, 8051 Zürich (CH)
(74) Representative: P&G Patent Germany

(56) References cited:
- WO-A1-2016/075492
- WO-A1-2016/099471
- US-A1- 2005 244 794
- Joe Min Moon ET AL: "Evaluation of Software Developed for Automated Segmentation of Digital Dental Models A THESIS SUBMITTED TO THE FACULTY OF THE GRADUATE SCHOOL OF THE UNIVERSITY OF MINNESOTA BY", , 29 February 2012 (2012-02-29), XP055310160, Retrieved from the Internet: URL:http://conservancy.umn.edu/bitstream/h andle/11299/123052/Moon_Joe Min_March2012.pdf?sequence=1&isAllowed=y [retrieved on 2016-10-12]
- SCHOENLY JOSHUA E ET AL: "Near-ultraviolet removal rates for subgingival dental calculus at different irradiation angles.", JOURNAL OF BIOMEDICAL OPTICS JUL 2011, vol. 16, no. 7, July 2011 (2011-07), pages 71404-01-71404-07, ISSN: 1560-2281
- DATABASE MEDLINE [Online] US NATIONAL LIBRARY OF MEDICINE (NLM), BETHESDA, MD, US; July 2011 (2011-07), SCHOENLY JOSHUA E ET AL: "Near-ultraviolet removal rates for subgingival dental calculus at different irradiation angles.", Database accession no. NLM21806250 & JOURNAL OF BIOMEDICAL OPTICS JUL 2011, vol. 16, no. 7, July 2011 (2011-07), page 71404, ISSN: 1560-2281

## Description

### FIELD OF THE INVENTION

Embodiments of the present invention relate to a method for determining dental plaque and a device for determining dental plaque.

In particular, the present invention describes an analytical procedure for identification and quantification of human biofilm (plaque) using a 3D model of a dentition or of at least one tooth of a dentition, wherein said 3D model may be obtainable with an intra-oral camera and analysis software.

### BACKGROUND OF THE INVENTION

Determination of plaque regions on teeth is frequently employed in clinical studies for proving the effectiveness of toothbrushes or other means and devices intended for removing oral biofilms, such as dental plaque. Determination of dental plaque may comprise detection and/or quantification of dental plaque.

Conventional procedures for recognition and quantitative analysis of plaque are usually based on a dental professional, e.g. a dentist, detecting stained areas of plaque.

The dental professional determines the presence and localizes the regions of plaque purely visually and marks them by means of a standardized plaque index. One example of a standardized plaque index is the so-called Rustogi Modified Navy Plaque Index (RMNPI).

A further example of a standardized plaque index is the so-called Turesky - Modification of the Quigley Hein Plaque Index (TQHPI).

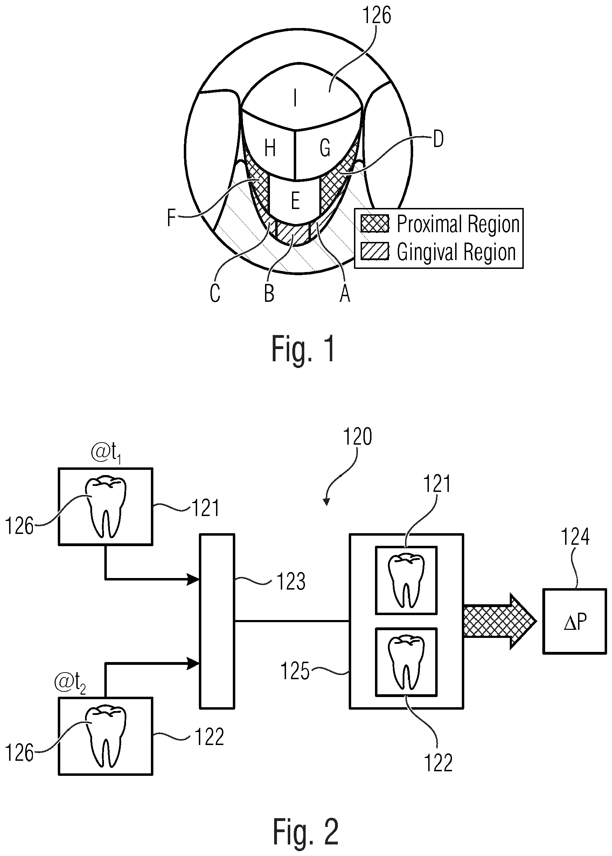

Figure 1 shows a schematic view of a tooth 126 on which the Rustogi Modified Navy Plaque Index (RMNPI) is applied. The dentist subdivides the tooth 126 into nine distinct regions A to I. Plaque on said tooth 126 may be visualized by staining, for example by means of particularly developed stain pills which have to be orally ingested. The dentist subjectively quantifies the occurrence of plaque in each sector A to I individually by visual inspection.

Alternatives employing planimetrical procedures or processing images of areas of the dentition using a 2D photographic procedure have also been described.

In most cases, this state-of-the-art plaque detection and quantification depends on the subjectivity and experience of the practitioner and its precision is limited due to the resolution of the selected index. Accordingly, the results of these commonly used plaque detection techniques may vary to a great extent amongst various practitioners. Due to the reliance on the subjectivity of the practitioner, even the results of different patients being examined by one and the same practitioner may vary to a great extent amongst each other. As mentioned above, determination, i.e. detection and quantification, of plaque regions on teeth may be employed in clinical studies for proving the effectiveness of toothbrushes or other means and devices intended for removing oral biofilms. However, due to the subjectivity of the practitioner the results may vary to a great extent and therefore an objective determination and quantization of plaque is rather complex and unreliable today. Accordingly, there is a desire to provide devices and methods which allow for objective and repeatable results in determining, i.e. detecting and/or quantifying, plaque without the drawbacks of today's methods.

A device for assessing removal rates for subgingival dental calculus is known from SCHOENLY JOSHUA E ET AL: "Near-ultraviolet removal rates for subgingival dental calculus at different irradiation angles. "JOURNAL OF BIOMEDICAL OPTICS JUL 2011, vol. 16, no. 7, July 2011, pages 71404-01-71404-07.

Document WO 2016/075492 A1 discusses a method of detecting and quantifying oral substrate in a subject in a trial to determine the efficacy of a test composition in reducing, preventing and/or treating oral substrate in the subject, wherein the subject is a companion animal, comprising the following steps: (i) obtaining one or more images of one or more teeth of a conscious test subject and a conscious control subject at the start of the trial (day 0) using an image taking device that is capable of detecting fluorescence, (ii) analysing the images to quantify the substrate coverage on each tooth of each subject at the day 0, (iii) administering the test subject with a test composition and the control subject with a control composition for the duration of the trial, (iv) obtaining one or more images of the same one or more teeth of step (i) of each of the test subject and the control subject at pre-determined intervals during the trial, (v) analysing the images to determine and quantify the substrate coverage and size on each tooth of each subject and compare with the images at the start of the trial and or at each stage of the trial, (vi) comparing the substrate coverage of the one or more teeth of the test subject and the control subject, and (vii) determining the efficacy of the test composition in reducing, preventing and/or treating oral substrate and/or periodontal diseases in the subject.

These desires are satisfied by an inventive method for determining dental plaque, the method having the features of independent claim 1, as well as by an inventive device for determining dental plaque, the device having the features of independent claim 9.

### SUMMARY OF THE INVENTION

A first aspect of the invention is concerned with a method for determining dental plaque as defined by claim 1.

A second aspect of the invention is concerned with a device for determining dental plaque as defined by claim 9.

### BRIEF DESCRIPTION OF THE DRAWINGS

In the following, embodiments of the present invention are described in more detail with reference to the figures, in which
- Fig. 1: shows a schematic view of a tooth having a RMNPI grid on its surface,
- Fig. 2: shows a block diagram of a device according to an embodiment,
- Fig. 3: shows a 3D-image displaying a maxilla comprising at least one tooth to be examined for dental plaque,
- Fig. 4: shows a further 3D-image displaying a maxilla on which image segmentation is to be applied for separating teeth from surrounding gingiva,
- Fig. 5: shows a further 3D-image displaying a maxilla on which image segmentation is to be applied for separating several teeth from each other,
- Fig. 6: shows a further 3D-image displaying a separated tooth with surrounding gingiva, wherein a grid has been transferred onto its surface,
- Fig. 7: shows a further 3D-image displaying a separated tooth, wherein a grid has been transferred onto its surface,
- Fig. 8: shows a further 3D-image displaying a maxilla, wherein a differential amount of plaque is visualized and a grid has been transferred onto its surfaces, and
- Fig. 9: shows a block diagram of a method according to an embodiment.

### DETAILED DESCRIPTION OF THE INVENTION

Equal or equivalent elements or elements with equal or equivalent functionality are denoted in the following description by equal or equivalent reference numerals.

Although some aspects will be described in the context of an apparatus or device, it is clear that these aspects also represent a description of the corresponding method, where a block or device corresponds to a method step or a feature of a method step. Analogously, aspects described in the context of a method or method step also represent a description of a corresponding block or item or feature of a corresponding apparatus or device.

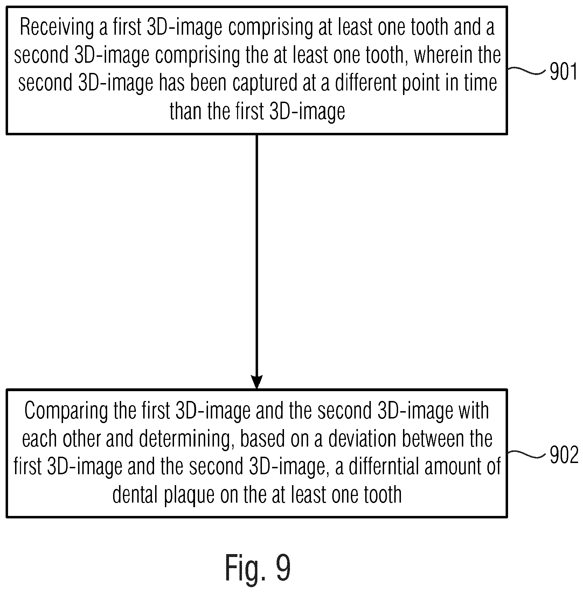

First of all, the inventive device shall be described with reference to the appended Figures 2 to 8. Afterwards, the inventive method, which may be executable with said inventive device, shall be described with reference to Figure 9. The explanations and descriptions of the device shall also serve as a representational description for the inventive method. In other words, everything that is explained herein with reference to the inventive device is also applicable to the inventive method and vice versa.

Figure 2 shows an example of a device 120 according to the present invention. The device 120 comprises an interface 123 for receiving a first 3D-image 21 comprising at least one tooth 126 and for receiving a second 3D-image 122 comprising the at least one tooth 126, wherein the second 3D-image 122 has been captured at a different point in time than the first 3D-image 121.

The device 120 further comprises an analysis unit 125 which is configured to compare the first 3D-image 121 and the second 3D-image 122 with each other and to determine, based on a deviation between the first 3D-image 121 and the second 3D-image 122, a differential amount 124 of dental plaque on the at least one tooth 126.

The 3D-images 121, 122 may be obtained by means of any 3D imaging device, for example an intra-oral scanner or an intra-oral camera. For example, a 3D intra-oral camera may be used for detecting the geometry of the tooth 126 and its surface textures.

While scanning the tooth 126, or even a full maxilla and/or mandible, combined data may be formed from the 3D geometry and information on the texture and combined in a file. Exemplary 3D data formats which may be used here include, e.g. Object (obj) and Polygon File Format (ply). These data may be exploited for quantifying plaque during one or more analysis stages following detection. Accordingly, a 3D-CAD method may, for instance, be used here for solving a dental problem, namely the detection and quantification of dental plaque.

In other words, the inventive device 120 for determining dental plaque comprises an interface 123 for receiving a first 3D-image 121 comprising at least one tooth 126 and a second 3D-image 122 comprising the at least one tooth 126. Dental plaque may also be referred to as biofilm and may reside on teeth and gingiva.

The first and second 3D-images 121, 122 may be obtained, for example, by means of an intra-oral camera or intra-oral scanner. These intra-oral imaging devices may particularly be useful when a tooth of a living being shall be examined as to dental plaque. In case of a human being, i.e. a person, being examined, dental plaque may also be referred to as human biofilm.

Apart from analyzing the existence or removal of human biofilm, the inventive principle may also be applied to in-vitro tests. For example, 3D laboratory color scanners may be used instead of the intra-oral imaging devices. These scanners provide the data required for analyzing plaque existence or removal from, for example, laboratory jaws coated with artificial plaque.

Independent of which imaging device may have captured the 3D images of the tooth 126, it is an aspect of the invention that the first and second 3D-images 121, 122 have been captured at a different point in time. For example, the first 3D-image 121 may have been captured from a tooth before a person has brushed his teeth. The second 3D-image may have been captured from the same tooth 126 but at a later point in time, e.g. after the person has brushed his teeth.

As mentioned above, the interface 123 of the inventive device 120 is configured to receive the first and second 3D-images 121, 122 from any imaging device. The inventive device 120 further comprises an analysis unit 125 which is configured to compare the first 3D-image 121 and the second 3D-image 122 with each other.

The analysis unit 125 is configured to detect any deviations between the tooth 126 being shown in the first 3D-image 121 in relation to the tooth 126 being shown in the second 3D-image 122. If a deviation may be detected between the first 3D-image 121 and the second 3D-image 122, the analysis unit 125 is to determine a differential amount of dental plaque on the at least one tooth 126 based on said deviation.

For example, if the first 3D-image 121 shows the tooth 126 in the above mentioned pre-brush situation, and the second 3D-image 122 shows the same tooth 126 in the above mentioned post-brush situation, then some extent of plaque may have been removed during the tooth brushing procedure. Accordingly, the second 3D-image 122 showing the post-brush situation of the tooth 126 may deviate from the first 3D-image 121 showing the pre-brush situation of the tooth 126.

The detected deviation between the first and the second 3D-images 121, 122 may be an indication for the amount of dental plaque that has been removed during the tooth brushing procedure that took place between capturing the first 3D-image 121 and the second 3D-image 122. For easier identification of dental plaque in any 3D-images, the dental plaque may be visualized by staining, using suitable means like plaque staining pills or the like, before capturing any 3D-images. However, depending on the precision and resolution of the imaging device, it may also be possible to directly detect dental plaque on the surface of a scanned tooth 126 without staining the tooth 126 beforehand. Accordingly, a differential dental plaque value may be directly obtainable from the 3D-images 121, 122. As mentioned above, the inventive device 120 may be fed with 3D-images 121, 122 which do not only show one single tooth 126 but even a full maxilla, or at least portions thereof, and/or a full mandilla, or at least portions thereof. Additionally or alternatively, the inventive device 120 may be fed with 3D images showing a full dentition including the maxilla and mandilla.

Thus, according to some embodiments, the interface 123 may be configured to receive the 3D-images, wherein the first 3D-image 121 shows at least a portion of a lower row of teeth comprising the at least one tooth 126 or an upper row of teeth comprising the at least one tooth 126, and wherein the second 3D-image 122 shows the portion of the lower or upper row of teeth comprising the at least one tooth 126, wherein the second 3D-image 122 has been captured at a different point in time than the first 3D-image 121.

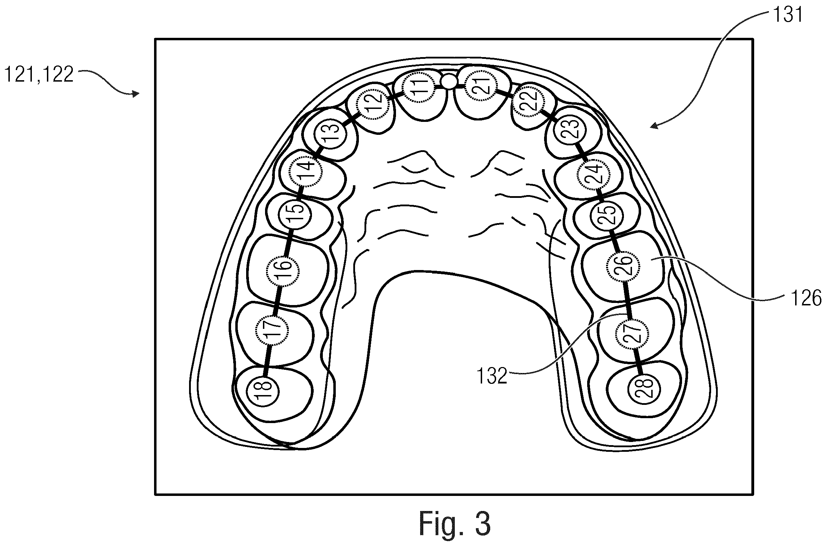

Figure 3 shows an exemplary 3D-image 121, 122 showing an upper row of teeth 131, also referred to as maxilla. This depicted 3D-image may be a first 3D-image 121 captured at a first point in time t₁, or a second 3D-image 122 captured at a second point in time t₂. The depicted teeth are numbered exemplarily in the so-called FDI scheme (FDI: Federation Dentaire Internationale), even though any other dental scheme may be used. For the purpose of the following description, tooth number 26 (FDI) is exemplarily picked here as representing the at least one tooth 126 to be examined for dental plaque.

In this step, which is merely optional, the dental status is determined. As can be seen in Figure 3, the 3D-image shows a maxilla 131 for determining the specific dentition. Any situations other than a full dentition may be registered. The approximate geometric centers of the teeth may be clicked on to mark them, which is depicted by means of the encircled FDI-numbers.

This merely optional step may be used in preparation for one or more subsequent steps which will be discussed in more detail below, for example for graphically separating (e.g. cropping) the at least one tooth 126 to be examined, or for transferring a predefined PIR (PIR: Plaque Index Region) grid to the specific tooth 126. Further optionally, a 3D snake 132 may be used for calculating the central spot. The step of determining the dental status may facilitate initial positioning of a separation surface, which will also be explained in detail below. However, should alternative means of tooth segmentation be used, this step may be omitted.

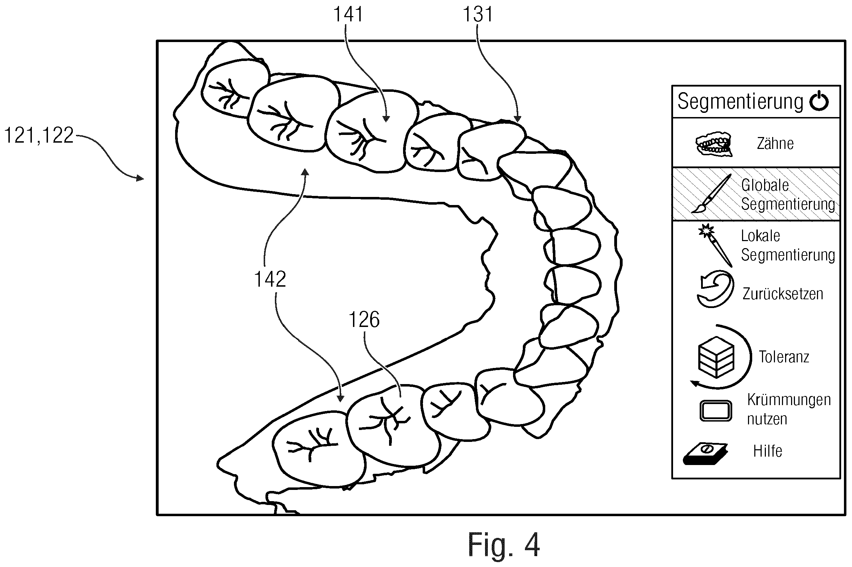

Figure 4 shows a further example of a 3D-image 121, 122 displaying a 3D-model of a maxilla 131. The 3D-image 121, 122 shows the upper row of teeth 141 comprising the at least one tooth 126 to be examined and gingiva 142 surrounding the teeth. In other words, the 3D-image 121, 122 comprises a first image region showing the at least one tooth 126 and a second image region showing the gingiva 142 surrounding the tooth 126. Said image region may also be referred to as a predetermined image section in the first and/or second 3D-images 121, 122.

The above mentioned image regions shall be understood as particular or predetermined areas within the 3D-image, for example given by certain coordinates and/or by different shade values and/or by different color values or the like.

According to an embodiment, the inventive device 120 may comprise an image segmentation unit which is configured to determine at least the first image region (e.g. showing the tooth 126) by exploiting image segmentation to separate the at least one tooth 126 from the surrounding gingiva 142 in the at least one of the first and second 3D-images 121, 122.

In other words, the image segmentation unit is configured to separate the at least one tooth 126 from the gingiva 142 in the 3D-image 121, 122. In other words, the at least one tooth 126 is graphically separated from the gingiva 142, which graphical separation may also be referred to as cropping. In the example shown in Figure 4, the image segmentation unit may be configured to separate or crop the entire upper row of teeth 141 from the surrounding gingiva 142 in the 3D-image 121, 122. After this step of image segmentation, the image segmentation unit may further be configured to display either the tooth 126 (or the entire upper row of teeth 141) or the gingiva 142 separately from each other.

Accordingly, the teeth 126, 141 may be separated from the gingiva 142 on the 3D model in the first and/or second 3D-images 121, 122. Various graphic and mathematical approaches may be employed. One possibility for such image segmentation may be based on selecting shades and/or hues and/or colors. Shades, hues or colors which may typically be associated with teeth (e.g. white/gray/yellow) are included by means of a selection process and gingival shades (e.g. redish/pinkish) may be selected for exclusion.

For example, the selected shades may be stored in an atlas in the form of a template. This so-called global segmentation may provide a rough tool for separating teeth 126, 141 and gingiva 142. Additionally or alternatively, a tolerance tool may determine which shades are recognized as being similar to the selected shade.

Additionally or alternatively, grooves in the 3D model displayed in the 3D-image 121, 122 may also be used for image segmentation. Where possible, the objective is to ensure that the edges of the segmented regions detected due to the shade are localized in the grooves in the 3D model.

Local segmentation functions according to the "magic wand principle" are known from various image processing tools. The shade is selected at the click point and all surrounding areas reachable from this point with a similar shade are also selected.

Here too, the function can be negated, i.e. shades can also be excluded. Thus, the selected regions are reduced rather than enlarged. When using this tool, the selected area can also be controlled with a tolerance slider. The groove tool is equipped with the same function as for global segmentation.

A "cleaning tool" may identify and automatically remove small holes in the image region or small spots which may, possibly, not be desirable. During reworking stage, the contrast of the image region shade may be increased. Areas within the image region may be allotted a shade and areas outside of the region may be displayed differently.

A margin smoother may smoothen the margins of the image region. This may involve generating a margin line and smoothing it. Following this, the region may be calculated anew from this margin line.

Furthermore, a so-called maximum "edge jump" may describe the maximum surface distance stretching from a region margin to a groove so that this groove may still be accepted. If the groove is further away, the region is not contracted or expanded toward the groove.

The tooth radius may be important for local segmentation. It describes the size of the tooth 126 and thus determines the area or image regions within which the segmentation takes effect. Areas further away from the click point may not be included in the segmentation region.

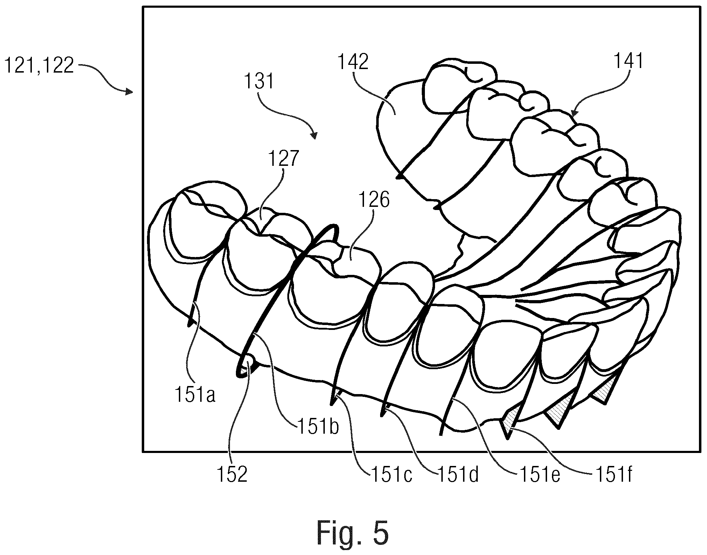

The above mentioned possibilities only describe exemplary types of image segmentation between the tooth 126 and gingiva 142 whereby other methods may also be feasible. In particular, methods which recognize a tooth 126 via a biogeneric approach shall be mentioned here. These procedures would also be suitable for the next stage as will be described in the following with reference to Figure 5.

Figure 5 shows an example of single tooth segmentation. Again, the depicted 3D-image 121, 122 shows a 3D-model of a maxilla 131 showing an upper row of teeth 141 and surrounding gingiva 142. The gingiva 142 is only exemplarily shown here. The gingiva 142 may also be separated by image segmentation as explained above, such that only the upper row of teeth 141 would be visible without the gingiva 142.

As can be seen, the upper row of teeth 141 comprises the at least one tooth 126 to be examined for dental plaque and at least one further tooth 127. In other words, the 3D-image 121, 122 comprises a first image region showing the at least one tooth 126 and a second image region showing the further tooth 127. As mentioned above, the image regions shall be understood as particular or predetermined areas within the 3D-image 121, 122, for example given by certain coordinates and/or by different shade values and/or by different color values or the like.

As shown in Figure 5, separation surfaces 151a, 151b, 151c, 151d and so on may be used for separating the at least one tooth 126 from the further tooth 127. These separation surfaces 151a, 151b, 151c, 151d separate the at least one tooth 126 from its surrounding, for example from the at least one further tooth 127.

As can be seen, the at least one tooth 126 resides adjacent to the further tooth 127. In this example, the separation surface 151b may separate both teeth 126, 127 from each other. Accordingly, the at least one tooth 126 may be separated from its one or more directly adjacent neighboring teeth. However, if the at least one tooth 126 does not have a directly adjacent neighboring tooth, for example if the at least one tooth 126 is a terminal tooth or the dentition comprises tooth gaps, then the separation surfaces 151b, 151c may nevertheless be used to separate the at least one tooth 126 from its surrounding. This can be done by exploiting image segmentation, as explained above.

The separation surfaces 151a, 151b, 151c, 151d are vertically arranged between two teeth 126, 127 being in contact with at least a portion of the approximal surface of the at least one tooth 126. In other words, the separation surfaces 151a, 151b, 151c, 151d may be vertically arranged such that they intersect the space in between two adjacent teeth, which may then be used to graphically separate these teeth from each other.

In other words, at least one of the first and the second 3D-images 121, 122 may comprise a first image region showing the at least one tooth 126 and a second image region showing at least one further tooth 127. According to an embodiment, the inventive device 120 may comprise an image segmentation unit being configured to determine the first and second image regions by exploiting image segmentation using at least one separation surface 151b between the first and second image regions to graphically separate the at least one tooth 126 from the at least one further tooth 127.

This procedure of separating the at least one tooth 126 from a further tooth 127 in the 3D-image may also be referred to as a single-tooth segmentation. With this so-called single tooth segmentation all of the teeth 141 may be separated from each other via separation surfaces 151a, 151b, 151c, 151d, as exemplarily described above with respect to the teeth 126, 127.

The positioning and alignment of the separation surfaces 151a, 151b, 151c, 151d may be controlled via sliding spheres 152. For example, software may automatically suggest where to place the separation surface 151a, 151b, 151c, 151d. Additionally or alternatively, domed separating surfaces may be necessary where simple flat sections are not sufficient, e.g. sloping or overlapping teeth.

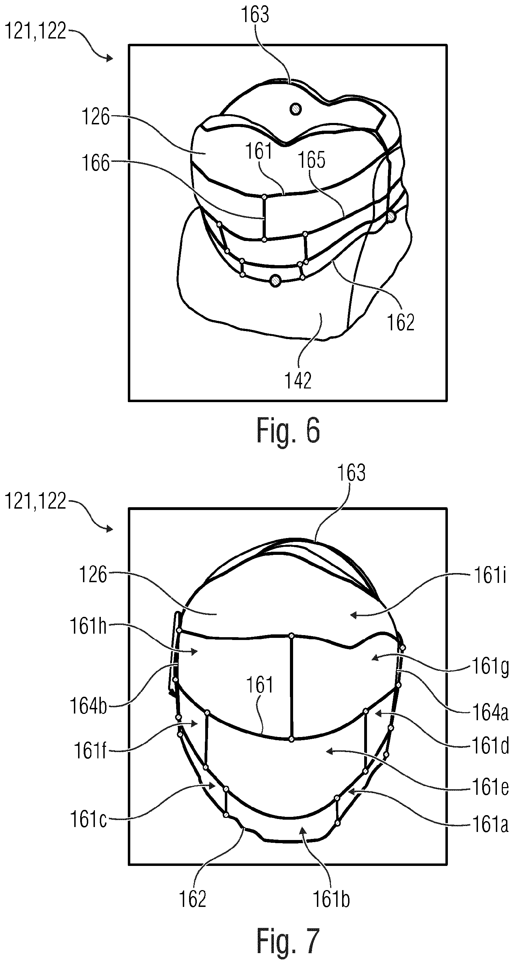

Further additionally or alternatively, 3D snakes may also be used for separating single teeth 126, 127 from each other and, in particular, generating tooth models which are also complete in the proximal region. A good quality of the tooth models is favorable for ensuring that PIR grids may be transferred precisely to the individual teeth, as will be explained in the following with reference to Figures 6 and 7. Furthermore, segmentation of individual teeth with the aid of 3D snakes can be enhanced by determining separating surfaces.

Figure 6 shows a graphically separated, or cropped, tooth 126 which has been separated by image segmentation as explained above. Just by way of example, parts of the surrounding gingiva 142 are depicted here.

However, the tooth 126 may also be displayed in the 3D-image without the gingiva 142 since the gingiva 142 may be separated from the tooth 126 by image segmentation as explained above. Such an example is depicted in Figure 7.

Both Figures 6 and 7 show a grid 161 that has been graphically transferred onto the tooth 126, more precisely onto the surface of the tooth 126. The grid 161 comprises multiple grid segments 161a, 161b, 161c, 161d, 161e, 161f, 161g, 161h, 161i.

The grid 161 may be a grid that can be used in conventional Dental Plaque Indices, and comprises at least one of the Rustogi Modified Navy Plaque Index (RMNPI) and a Turesky-Modification of the Quigley Hein Plaque Index /TQHPI).

The grid 161 shown in Figures 6 and 7 is, for instance, a grid that can be used in the Rustogi Modified Navy Plaque Index (RMNPI), as discussed above with reference to Figure 1.

As can best be seen in Figure 7, the grid 161 may comprise at least nine different grid segments 161a, 161b, 161c, 161d, 161e, 161f, 161g, 161h, 161i which correspond to the RMNPI index regions A to I shown in Figure 1. Thus, in this example, the grid segments 161a to 161i may also be referred to as index regions, and the grid 161 may also be referred to as a PIR grid.

In such a PIR grid 161, the differential amount of dental plaque on the at least one tooth 126 may be determined based on the Index Regions 161a to 161i. For example, each index region 161a to 161i may be separately determined as to an amount of dental plaque within said index region 161a to 161i. Optionally, these individual results of the determined amount of dental plaque within each index region 161a to 161i may be combined to obtain a result of the total amount of dental plaque of the entire tooth 126, or even of the entire jaw or dentition.

The grid 161 may be transferred onto the surface of the tooth 126 by the image computation unit based on a segmentation line 162 being graphically depicted or drawn and thus visible within the 3D-image 121, 122. This separation line 162 may, for example, be a segmentation line that may have been drawn in order to separate the tooth 126 from the surrounding gingiva 142, as explained above with reference to Figure 4. In other words, the grid 161 may be transferred onto the surface of the tooth 126 by the image computation unit by anchoring at least a portion of the grid 161 on said segmentation line. For example, as shown in Figure 6, a lowermost portion of the grid 161 has been anchored on the segmentation line separating the at least one tooth 126 from the surrounding gingiva 142.

Accordingly, the selected segmentation line 162 may serve as a basis line, wherein the grid 161 can be snapped onto said basis line 162. Accordingly, the image computation unit snaps the grid 161 onto the surface on the tooth 126 by aligning at least one line of the grid 161 with the basis line 162. In this example, the image computation unit transfers the grid 161 onto the surface of the tooth 126 by aligning the lower line of the grid 161 with the segmentation line 162 for separating the tooth 126 from the gingiva 142.

According to a further embodiment, the grid 161 may be based on a previously generated standard grid having been generated on a standard model tooth, wherein the image computation unit is configured to adjust the dimensions of the standard grid to the surface of the at least one tooth 126. In this way, the standard grid may be exactly snapped, fitted or transferred onto the surface of the tooth 126 and can then be used as a grid 161 as mentioned above.

In other words, the grid 161 described in the Plaque Index for defining the zones A to I on the at least one tooth 126 may be generated once only on a standard jaw using graphics tools and can be transferred to every 3D dentition model displayed in the first and/or second 3D-images 121, 122. The segmentation line that may have been generated previously between the tooth 126 and gingiva 142 may be used for this purpose. Optionally, a parametrization may be used for transferring the Plaque Index from a standardized jaw to a specific jaw, i.e. when transferring the standard grid to the grid 161 used for the particular tooth 126. When doing so, the image computation unit is configured to ensure that transferring the PIR grid maintains the relative index dimensions, i.e. they are not distorted when scaled.

Thanks to the free design options for a PIR grid 161, it is possible to analyze surfaces not previously reflected in commonly used indices, e.g. occlusal surfaces. Therefore, a cusp line 163 may be drawn to mark the occlusal region of the tooth 126.

Furthermore, lines 164a, 164b (Figure 7) in the interdental region may separate the exterior surface of the tooth 126 from its interior. Further lines, such as horizontal lines 165 and vertical lines 166 (Figure 6), may be drawn to create the grid 161, depending on the selected Plaque Index. The thus created index partial surfaces, i.e. index regions 161a to 161i, may be provided with nomenclature, for instance A to I as used in the RMNPI. Defined partial surfaces 161a to 161i may be shown full size and their value of plaque amount may be displayed.

As mentioned before, the first and second 3D-images 121, 122 may display the same content, e.g. one and the same tooth 126, an upper or lower row of teeth, or even a full dentition. The image computation unit may superimpose the first 3D-image 121 and the second 3D-image 122. As mentioned before, at least the first 3D-image 121 may display the tooth 126 having the grid 161 transferred on its surface. Again, the second 3D-image 122 may also display the same tooth 126 but without having a grid 161 transferred thereon. By superimposing this first 3D-image 121 with the second 3D-image 122, the grid 161 of the first 3D-image 121 may be easily transferred to the second 3D-image 122. In other words, the grid 161 that resides on the surface of the tooth 126 displayed in the first 3D-image 121 may be directly transferred onto the surface of the tooth 126 being displayed in the second 3D-image 122. This can be done by aligning the tooth 126 of the first 3D-image 121 with the tooth 126 of the second 3D-image 122, e.g. to move and/or rotate the tooth 126 such that the first and second 3D-images 121, 122 fit together. When the tooth 126 of the first 3D-image 121 is fitted to the size of the tooth 126 in the second 3D-image 122, then the grid 161 may be easily transferred from the first 3D-image 121 into the second 3D-image 122 such that the grid 161 of the tooth 126 of the first 3D-image 121 fits the size of the tooth 126 of the second 3D-image 122. Accordingly, the grid 161 of the first 3D-image 121 can be reused such that there is no need to create a complete new grid 161 in the second 3D-image 122 which may be time consuming.

According to such an embodiment, the image computation unit may be configured to superimpose the first 3D-image 121 with the second 3D-image 122, wherein the first 3D-image 121 comprises the at least one tooth 126 having the grid 161 transferred onto its surface and the second 3D-image 122 comprises the at least one tooth 126 but without a grid, and wherein the image computation unit is further configured to align the at least one tooth 126 of the first 3D-image 121 with the at least one tooth 126 of the second 3D-image 122 and to transfer the grid 161 of the at least one tooth 126 of the first 3D-image 121 onto the surface of the at least one tooth 126 of the second 3D-image 122.

As mentioned before, the first and second 3D-images 121, 122 are captured at different points in time. For example, the first and second 3D-images 121, 122 may be used to determine an amount of dental plaque removal between the first and second points in time. Therefore, the first 3D-image 121 may be captured at a first point in time t₁ at which the tooth 126 to be examined has not yet been brushed. The second 3D-image 122 may be captured at a later point in time t₂, for example after the tooth 126 to be examined has been brushed. Accordingly, the first point in time t₁ may be referred to as a pre-brush situation while the second point in time t₂ may be referred to as a post-brush situation.

The differential amount of dental plaque that is discussed herein may represent the difference between a first amount of dental plaque at a first time instant and a second amount of dental plaque at a second time instant. Accordingly, the term differential amount of dental plaque may, for instance, represent an amount of added or removed dental plaque. In the latter case, the differential amount of dental plaque may be expressed as a plaque removal value.

Coming back to the example above, a pre-brush grid 161 may be transferred to the post-brush situation by superimposing the individual teeth 126 from both dentition models. As discussed above, this may be done by superimposing the individual teeth 126 from both 3D-images 121, 122 geometrically so that they occupy the same space. Thus, superimposing results in displacement and rotation of every single tooth in the pre-brush jaw with the aid of which the individual tooth 126 can be transformed to the same tooth 126 in the post-brush jaw. By applying these transformations also to the PIR grid 161 of the pre-brush situation it can be superimposed over the post-brush situation and thus used there directly.

By superimposing the first and the second 3D-images 121, 122 the differential amount of dental plaque on the at least one tooth 126 may be determined. For example, the first 3D-image 121 shows the tooth 126 at the pre-brush situation and the second 3D-image 122 shows the tooth 126 at the post-brush situation, as mentioned above.

When superimposing the first and second 3D-images 121, 122 a delta value, i.e. a differential value, between the first and second 3D-images 121, 122 may be detected. This differential value may represent a difference between a first amount of dental plaque on the tooth 126 of the first 3D-image 121 (e.g. pre-brush) compared to a second amount of dental plaque on the tooth 126 in the second 3D-image 122 (e.g. post-brush).

This delta or differential value may therefore be considered as the amount of plaque that has been removed between the two time instants t₁ and t₂, i.e. between the capturing of the first 3D-image 121 (e.g. pre-brush) and the capturing of the second 3D-image 122 (e.g. post-brush). It may therefore also be referred to as a plaque removal value.

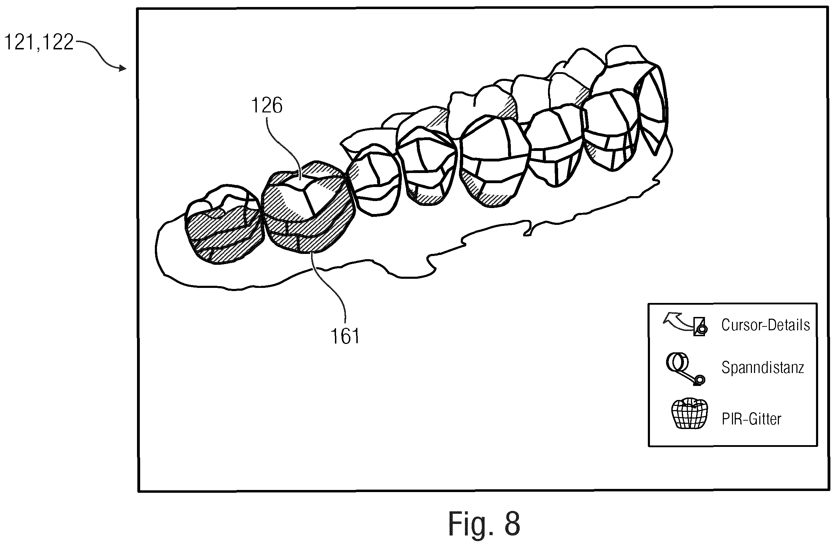

An exemplary scenario is depicted in Figure 8. This example shows the superimposition of a first and a second 3D-image 121, 122 in which a jaw is displayed. As can be seen, the at least one tooth 126 has a grid 161 transferred onto its surface. Further teeth of this jaw also have grids transferred onto their respective surfaces. The grids may correspond to the grids 161 discussed above with reference to Figures 6 and 7. For instance, the grid 161 may be a PIR grid of an RMNP Index.

As can be seen in Figure 8, the shaded regions highlighted in hatched lines represent the above mentioned delta plaque value, i.e. the amount of removed plaque when comparing the first 3D-image 121 with the superimposed second 3D-image 122. The amount of removed plaque can be given in terms of mm² or in percent, for example, which may be referred to as the plaque removal value.

According to embodiments, this plaque removal value may be calculated for each grid segment 161a to 161i individually. As required by the index, i.e. optionally, the partial surfaces of the grid segments 161a to 161i may be combined to form a total result indicating a total plaque removal value for the at least one tooth 126, or maybe even for the entire mandible, maxilla or dentition, respectively.

In other words, for a quantification of dental plaque or dental plaque removal, index grids 161 may be mapped onto the scanned teeth within at least one of the first and second 3D-images 121, 122. The difference or deviations between the pre-brush und post-brush situations may be used to calculate the plaque removal value for each index partial surface 161a to 161i. As mentioned above, these surface values can be quoted in mm² or %. As required by the index, the partial surfaces may be combined to form a total result.
Thus, according to an embodiment, the image computation unit may be configured to superimpose the first 3D-image 121 and the second 3D-image 122 and to determine a differential image value between the first and second 3D-images 121, 122. Said differential image value may indicate a deviation between the first and second 3D-images 121, 122, for example a deviation in the displayed amount of plaque on the at least one tooth 126 to be examined. Accordingly, the magnitude of said differential image value may represent the differential amount of dental plaque on the at least one tooth 126, e.g. the above mentioned plaque removal value.

Still with reference to Figure 8, the inventive device 120 may also be configured to display not only the above mentioned differential amount of dental plaque, i.e. the plaque removal value, but it may also be configured to display in the first and second 3D-images 121, 122 the actual current amount of plaque which resides on the at least one tooth 126 at that time instant at which the respective 3D-image 121, 122 was captured. In other words, the 3D-images 121, 122 may display the current amount of dental plaque that resides on the tooth 126. This may be helpful for a practitioner or the like to determine the current situation of dental plaque on the tooth 126. For example, Figure 8 could be a 3D-image 121, 122 in which the shaded regions in hatched lines represent the amount of dental plaque which is currently present at the tooth 126 at the time of capturing said 3D-image 121, 122 instead of representing the above discussed plaque removal value.

Figure 9 shows a schematic diagram of an inventive method for determining dental plaque.

In block 901, a first and a second 3D-image 121, 122 is received, the first 3D-image 121 comprising at least one tooth 126 and the second 3D-image 122 also comprising the at least one tooth 126, wherein the second 3D-image 122 has been captured at a different point in time than the first 3D-image 121.

In block 902, the first 3D-image 121 and the second 3D-image 122 are compared with each other. Furthermore, a differential amount of dental plaque on the at least one tooth 126 is determined based on a deviation between the first 3D-image 121 and the second 3D-image 122.

The above described determination, i.e. detection and/or quantification, of dental plaque in the 3D-images 121, 122 may further be improved by staining the at least one tooth 126 before capturing the respective 3D-image 121, 122.

In case that an intra-oral camera or the like may be used, the resolution of the analysis can be increased considerably and is not limited by the definition of an established index.

Thanks to the improved resolution expected with intra-oral cameras in future, it will be possible in the medium-term to carry out volumetric evaluation instead of surface evaluation. This would eliminate the limitations in current 2D surface indexes. This would, for example, provide for analysis of the removal of different types of old plaque. Staining the plaque would no longer be required.

Apart from analyzing removal of human biofilm (e.g. dental plaque), this procedure may also be applied to in-vitro tests. 3D laboratory color scanners may be used instead of an intra-oral camera. They provide the data required for analyzing plaque removal from, for example, laboratory jaws coated with artificial plaque.

It may also be conceivable that, based on this new method, further higher resolution plaque indexes may become established.

In future, it may also be conceivable that it may be possible to automate manual selection such as shade determination by integrating machine learning algorithms.

Summarizing, the herein described devices, methods, uses and computer programs may allow for a detection and/or determination and/or quantification of material, for example a biofilm, such as dental plaque, residing on at least one tooth 126. Currently residing material may be detected and/or determined and/or quantified. Additionally or alternatively, a differential amount of said material may be detected and/or determined and/or quantified.

For example, a determination of plaque regions on teeth may be employed in clinical studies for proving the effectiveness of toothbrushes or other means and devices intended for removing oral biofilms.

This inventive concept may involve scanning a jaw several times, for instance at least twice, with an intra-oral camera and storing the geometries and textures thus recorded. During a first stage stained plaque on the teeth may be scanned together with the tooth geometry (so-called pre-brush assessment). A second stage may involve re-staining and scanning the plaque remaining after brushing (so-called post-brush assessment). The difference between residual plaque (stage 2) and initial plaque (stage 1) may be taken as a measurement for determining the plaque-removal performance of the toothbrush, tooth-cleaning material or device. Pre- and post-brush data may also be collected in the same manner in order to produce statements on compliance, the dynamic course of plaque accumulation and its removal or the combined effects of mechanical and chemical components in oral hygiene.

Quantification of cleaning-performance may, for instance, be carried out by determining the difference in surface area of the sum of all 3D partial tooth surfaces with initial plaque and residual plaque in mm² or in %, or, as mentioned above, by using a dental plaque index.

In order to map a plaque index on 3D tooth surfaces several preparatory measures may be employed, as discussed in detail above. Said measures may be summarized in the following five steps:
1. Determination of the pre-brush situation using a 3D model of the dentition.
2. Segmentation of the dentition into Plaque Index Regions (PIR).
   a. Determination of the dental status.
   b. Segmentation of the dentition/gingiva.
   c. Segmentation of the individual teeth (fabricate a model of every single tooth).
   d. Transfer a standard PIR grid to every single tooth.
3. Determination of the post-brush situation using a 3D model of the dentition.
4. Direct transferal of the segmentation from stage 2 to the post-brush situation.
   a. Automatic determination of the plaque index for the pre and post-brush image.

Furthermore, Taking an initial scan of the thoroughly cleaned jaw (so-called baseline assessment - BL) may enhance the quality of the analysis but is not essential for the procedure.

As mentioned above, the first and second 3D-images 121, 122 comprises the at least one tooth 126 to be examined. However, apart from the at least one tooth 126, further details may be shown in the first and second 3D-images 121, 122. For example, the first 3D-image 121 may show at least a portion of a lower row of teeth comprising the at least one tooth 126 and/or an upper row of teeth comprising the at least one tooth 126, and the second 3D-image 122 may show the portion of the lower or upper row of teeth comprising the at least one tooth 126, wherein the second 3D-image 122 has been captured at a different point in time than the first 3D-image 121. Accordingly, the interface 123 of the inventive device 120 may be configured to receive a 3D-image 121, 122 of a complete lower jaw, or at least portions thereof, including the at least one tooth 126. Additionally or alternatively, the interface 123 of the inventive device 120 may be configured to receive a 3D-image 121, 122 of a complete upper jaw, or at least portions thereof, including the at least one tooth 126. Further additionally or alternatively, the interface 123 of the inventive device 120 may be configured to receive a 3D-image 121, 122 of a complete dentition, or at least portions thereof, including the at least one tooth 126. In other words, the 3D-images 121, 122 may show a 3D view of a dentition where a practitioner may select areas of interest therefrom, for example the at least one tooth 126, additional teeth, or even all available teeth in the dentition. This has the advantage that the practitioner does not have to scan each tooth 126 individually but he may scan the complete dentition and selectively choose individual teeth afterwards within the 3D-images which selected teeth may be examined as to dental plaque.

For example, at least one of the first and second 3D-images 121, 122 may comprise a first image region showing the at least one tooth 126 and a second image region showing gingiva 142 surrounding the at least one tooth 126. According to an example, the inventive device 120 comprises an image segmentation unit being configured to determine at least the first image region by exploiting image segmentation to separate the at least one tooth 126 from the surrounding gingiva 142 in the at least one of the first and second 3D-images 121, 122. For example, if the imaging device may have captured gingiva 142 surrounding the tooth 126 to be examined, then the gingiva 142 may be displayed within the captured 3D-image 121, 122. The image segmentation unit is configured to extract or separate the displayed gingiva 142 from the displayed tooth 126 within the 3D-image 121, 122 by means of image segmentation. Accordingly, the tooth 126 may be separated from the gingiva 142 in the respective 3D-image 121, 122. For example, the 3D-image 121, 122 may show the at least one tooth 126 to be examined without displaying the gingiva 142 anymore.

For example, at least one of the first and the second 3D-images 121, 122 may comprise a first image region showing the at least one tooth 126 and a second image region showing at least one further tooth 127. In other words, two or more teeth 126, 127 may be displayed in the 3D-image 121, 122. According to an example, the inventive device 120 comprises an image segmentation unit being configured to determine said first and second image regions by exploiting image segmentation using a separation surface between the first and second image regions to separate the at least one tooth 126 from the at least one further tooth 127. In other words, if two or more teeth 126, 127 are displayed within the 3D-image 121, 122, the image segmentation unit is configured to arrange at least one segmentation surface between the two teeth 126, 127. The two teeth 126, 127 may be two adjacent teeth wherein the segmentation surface may be arranged in the interdental space between these two teeth 126, 127. As a result, the two teeth 126, 127 have been segmented and graphically separated, or cropped, as two individual objects and may therefore be selected individually within the 3D-image 121, 122.

According to yet a further example, the inventive device 120 may comprise an image computation unit being configured to transfer, in at least the first 3D-image 121, a grid 161 comprising multiple grid segments 161a-161i onto the surface of the at least one tooth 126. The grid 161 may be registered to or fitted onto the surface of the tooth 126. Accordingly, the lines of the grid 161 preferably follow the shape of the surface of the at least one tooth 126. The grid 161 may be fitted onto the entire surface of the tooth 126 or onto at least a portion of the surface of the tooth 126. In particular, the grid 161 may be fitted onto the lateral surfaces (mesial, labial, distal, approximal, palatinal, bukkal) of the tooth and optionally also onto the top (occlusal) surface, i.e. the tooth crown or cusps of the tooth 126.

For example, the grid segments of the grid 161 may represent Index Regions of a Dental Plaque Index comprising at least one of a Rustogi Modified Navy Plaque Index (RMNPI) and a Turesky-Modification of the Quigley Hein Plaque Index (TQHPI). According to such an example, the analysis unit may be configured to determine the differential amount of dental plaque on the at least one tooth 126 based on the Index Regions of the Dental Plaque Index. In other words, the analysis unit may exploit an RMNP Index or an TQHP Index for determining the actual current and/or differential amount of dental plaque on the tooth 126. The respective index may comprise several index regions 161a-161i that may be represented by the single grid 161 segments which have been fitted onto the surface of the tooth 126. The determination of the amount of plaque may be computed by the image computation unit exploiting algorithms using image recognition.

According to an example, the image computation unit may be configured to determine a differential amount of dental plaque in each grid segment 161a-161i individually. Optionally the image computation unit may combine the differential amount of each grid segment 161a-161i to obtain the total differential amount of dental plaque of the at least one tooth 126. In other words, the image computation unit may detect dental plaque on the tooth 126 in each index region, i.e. grid segment 161a-161i, individually. Additionally or alternatively, the image computation unit may determine the amount of plaque in each index region, i.e. grid segment 161a-161i, individually. This may be executed by determining how much of the grid segment 161a-161i is covered by dental plaque. The result may be output in terms of a metric of the covered surface, for example in terms of mm², and/or in terms of a percentage of the covered surface, i.e. how much percent of the considered grid segment 161a-161i, i.e. index region, is covered with plaque. The image computation unit may be configured to determine the differential amount of each grid segment 161a-161i individually. For example, when using an RMNP Index, the image computation unit may be configured to determine the differential amount of nine grid segments 161a-161i, i.e. nine index regions. Optionally, the image computation unit may be configured to combine (for example to add) the result of each individual grid segment 161a-161i, or at least of those grid segments in which plaque has been detected, in order to obtain an overall result of an amount of plaque for the whole tooth 126.

According to an example, the image computation unit may be configured to transfer the grid 161 based on a segmentation line drawn within the at least first 3D-image 121, the segmentation line for separating a first image region from a second image region. For example, the first image region may display the tooth 126 while the second image region may display gingiva 142 surrounding the tooth 126. In this example, the image segmentation line separates the tooth 126 from the surrounding gingiva 142. Accordingly, this particular segmentation line may serve as a basis on which the grid 161 is fitted or aligned. In other words, at least one grid line 161a-161i coincides with the segmentation line such that the grid 161 is snapped or fixed to said segmentation line.

For example, the image computation unit is configured to use a grid 161 which is based on a previously generated standard grid having been generated on a standard model tooth, wherein the image computation unit is configured to adjust the dimensions of the standard grid to the surface of the at least one tooth 126. In other words, an universal or standard grid may be available which has to be fitted onto the tooth 126 to be examined. The tooth 126 to be examined may usually not be the same tooth as the one on which the standard grid has been previously formed. Therefore, the image computation unit is configured to scale the standard grid such that it fits onto the surface of the tooth 126 to be examined. According to yet a further example, the image computation unit may be configured to superimpose the first 3D-image 121 with the second 3D-image 122, wherein the first 3D-image 121 comprises the at least one tooth 126 having the grid 161 transferred onto its surface and the second 3D-image 122 comprises the at least one tooth 126 but without a grid, and wherein the image computation unit is further configured to align the at least one tooth 126 of the first 3D-image 121 with the at least one tooth 126 of the second 3D-image 122 and to transfer the grid 161 of the at least one tooth 126 of the first 3D-image 121 onto the surface of the at least one tooth 126 of the second 3D-image 122. In other words, it may be sufficient to transfer the grid 161 only onto the surface of the tooth 126 being displayed in the first 3D-image 121. The tooth 126 being displayed in the second 3D-image 122, however, does not yet have a grid 161 transferred onto its surface. The image computation unit is configured to superimpose the first 3D-image 121 with the second 3D-image 122 and to align the tooth 126 displayed in the first 3D-image 121 with the tooth 126 displayed in the second 3D-image 122. Alignment of the tooth 126 in the first 3D-image 121 with the tooth 126 in the second 3D-image 122 may be done by moving and/or rotating the tooth 126 in the first 3D-image 121 relative to the tooth 126 in the second 3D-image 122. This may also be referred to as rigid body moving. As an optional step of alignment, the tooth 126 in the first 3D-image 121 may be scaled in size relative to the tooth 126 in the second 3D-image 122. At the same time, the grid 161 that resides on the surface of the tooth 126 displayed in the first 3D-image 121 is aligned by the same amount. Accordingly, if the tooth 126 displayed in the first 3D-image 121 is aligned with the tooth 126 displayed in the second 3D-image 122, then the grid 161 is also aligned so that it can directly be transferred onto the surface of the tooth 126 displayed in the second 3D-image 122 which did not comprise any grid before. By this easy step, there is no need of modelling a grid 161 for the tooth 126 in both the first and the second 3D-image 121, 122, but to reuse the grid 161 of the first 3D-image 121 in the second 3D-image 122. According to yet a further example, the image computation unit is configured to superimpose the first 3D-image 121 and the second 3D-image 122 and to determine a differential image value between the first and second 3D-images 121, 122, wherein the magnitude of the differential image value represents the differential amount of dental plaque on the at least one tooth 126. In other words, by superimposing both the first and the second 3D-images 121, 122 a deviation may be detected. This deviation between the first and second 3D-images 121, 122 may be represented by a differential image value. This deviation, i.e. the differential image value, may represent dental plaque that has been removed between capturing the first 3D-image 121 and capturing the second 3D-image 122. For example, between these two time instances of capturing the first and second 3D-images 121, 122 a person may have brushed his teeth in order to remove at least a certain amount of dental plaque. In other words, the differential image value represents the differential amount of dental plaque on the at least one tooth 126.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus. Some or all of the method steps may be executed by (or using) a hardware apparatus, like for example, a microprocessor, a programmable computer or an electronic circuit. In some embodiments, one or more of the most important method steps may be executed by such an apparatus.

Depending on certain implementation requirements, embodiments of the invention can be implemented in hardware or in software or at least partially in hardware or at least partially in software. The implementation can be performed using a digital storage medium, for example a floppy disk, a DVD, a Blu-Ray, a CD, a ROM, a PROM, an EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may for example be stored on a machine readable carrier.

Other embodiments comprise the computer program for performing one of the methods described herein, stored on a machine readable carrier.

In other words, an example of the disclosure is, therefore, a computer program having a program code for performing one of the methods described herein, when the computer program runs on a computer.

A further example is, therefore, a data carrier (or a digital storage medium, or a computer-readable medium) comprising, recorded thereon, the computer program for performing one of the methods described herein. The data carrier, the digital storage medium or the recorded medium are typically tangible and/or non-transitory.

A further embodiment of the inventive method is, therefore, a data stream or a sequence of signals representing the computer program for performing one of the methods described herein. The data stream or the sequence of signals may for example be configured to be transferred via a data communication connection, for example via the Internet.

A further embodiment comprises a processing means, for example a computer, or a programmable logic device, configured to or adapted to perform one of the methods described herein.

A further embodiment comprises a computer having installed thereon the computer program for performing one of the methods described herein.

A further example comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

The apparatus described herein may be implemented using a hardware apparatus, or using a computer, or using a combination of a hardware apparatus and a computer.

The methods described herein may be performed using a hardware apparatus, or using a computer, or using a combination of a hardware apparatus and a computer.

The above described embodiments are merely illustrative for the principles of the present invention. It is understood that modifications and variations of the arrangements and the details described herein will be apparent to others skilled in the art. It is the intent, therefore, to be limited only by the scope of the impending patent claims and not by the specific details presented by way of description and explanation of the embodiments herein.

Furthermore, the dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A computer-implemented method for determining dental plaque, the method comprising the steps of:
receiving a first 3D-image (121) comprising at least one tooth (126) and a second 3D-image (122) comprising the at least one tooth (126), wherein the second 3D-image (122) has been captured at a different point in time than the first 3D-image (121),
comparing the first 3D-image (121) and the second 3D-image (122) with each other and determining, based on a deviation between the first 3D-image (121) and the second 3D-image (122), a differential amount of dental plaque on the at least one tooth (126), and
further comprising the step of transferring, within at least the first 3D-image (121), a grid (161) comprising multiple grid segments (161a - 161i) onto the surface of the at least one tooth (126);
wherein the grid segments (161a - 161i) represent Index Regions of a Dental Plaque Index comprising at least one of a Rustogi Modified Navy Plaque Index (RMNPI) and a Turesky-Modification of the Quigley Hein Plaque Index (TQHPI), and wherein the differential amount of dental plaque on the at least one tooth (126) is determined based on the Index Regions of the Dental Plaque Index.

2. The method in accordance with claim 1, wherein at least one of the first and second 3D-images (121, 122) comprises a first image region showing the at least one tooth (126) and a second image region showing gingiva (142) surrounding the at least one tooth (126), and wherein the method comprises the step of determining the first image region in at least one of the first and second 3D-images (121, 122) by exploiting image segmentation to separate the at least one tooth (126) from the surrounding gingiva (142).

3. The method in accordance with one of claims 1 or 2, wherein at least one of the first and the second 3D-images (121, 122) comprises a first image region showing the at least one tooth (126) and a second image region showing at least one further tooth (127), wherein the method further comprises the step of determining the first and second image regions in at least one of the first and the second 3D-images (121, 122) by exploiting image segmentation using a separation surface (51b) between the first and second image regions to separate the at least one tooth (126) from the at least one further tooth (127).

4. The method in accordance with one of claims 1 to 3, wherein the step of determining the differential amount of dental plaque on the at least one tooth (126) comprises determining a differential amount of dental plaque in each grid segment (161a - 161i) individually, and combining the determined differential amounts of dental plaque in each grid segment (161a - 161i) to obtain a total differential amount of dental plaque of the entire grid (161) transferred onto the surface of the at least one tooth (126).

5. The method in accordance with one of claims 1 to 4, wherein the step of transferring the grid (161) onto the surface of the at least one tooth (126) comprises transferring the grid (161) onto the surface of the at least one tooth (126) by anchoring at least a portion of the grid (161) on a segmentation line drawn within the at least first 3D-image (121), the segmentation line for separating a first image region from a second image region in the at least first 3D-image (121).

6. The method in accordance with one of claims 1 to 5, wherein the grid (161) is based on a previously generated standard grid having been generated on a standard model tooth, wherein the method further comprises the step of adjusting the dimensions of the standard grid to the dimensions of the at least one tooth (126) and to apply the thus adjusted standard grid onto the surface of the at least one tooth (126).

7. The method in accordance with one of claims 1 to 6, further comprising the step of superimposing the first 3D-image (121) with the second 3D-image (122), wherein the first 3D-image (121) comprises the at least one tooth (126) having the grid (161) transferred onto its surface and the second 3D-image (122) comprises the at least one tooth (126) but without a grid, and aligning the at least one tooth (126) of the first 3D-image (121) with the at least one tooth (126) of the second 3D-image (122) and transferring the grid (161) of the at least one tooth (126) of the first 3D-image (121) onto the surface of the at least one tooth (126) of the second 3D-image (122).

8. The method of one of the preceding claims, further comprising the step of superimposing the first 3D-image (121) with the second 3D-image (122) and determining a differential image value between the first and second 3D-images (121, 122), wherein the magnitude of the differential image value represents the differential amount of dental plaque on the at least one tooth (126).

9. A device (120) configured for determining dental plaque, the device (120) comprising:
an interface (123) configured for receiving a first 3D-image (121) comprising at least one tooth (126) and a second 3D-image (122) comprising the at least one tooth (126), wherein the second 3D-image (122) has been captured at a different point in time than the first 3D-image (121),
an analysis unit (125) which is configured to compare the first 3D-image (121) and the second 3D-image (122) with each other and to determine, based on a deviation between the first 3D-image (121) and the second 3D-image (122), a differential amount (124) of dental plaque on the at least one tooth (126), and
an image computation unit being configured to transfer, within at least the first 3D-image (121), a grid (161) comprising multiple grid segments (161a to 161i) onto the surface of the at least one tooth (126), wherein the grid segments (161a to 161i) represent Index Regions of a Dental Plaque Index comprising at least one of a Rustogi Modified Navy Plaque Index (RMNPI) and a Turesky-Modification of the Quigley Hein Plaque Index (TQHPI), and wherein the differential amount of dental plaque on the at least one tooth (126) is determined based on the Index Regions of the Dental Plaque Index.

10. The device (120) of claim 9, wherein the image computation unit is configured to superimpose the first 3D-image (121) with the second 3D-image (122) for determining a differential image value representing a differential amount of dental plaque in each grid segment (161a to 161i) individually, and to combine the differential image values of each grid segment (161a to 161i) to obtain a total differential image value representing a total differential amount of dental plaque of the at least one tooth (126), wherein the magnitude of the total differential image value represents the total differential amount of dental plaque on the at least one tooth (126).

## Patentansprüche

1. Computerimplementiertes Verfahren zum Bestimmen von Zahnbelag, wobei das Verfahren die Schritte umfasst:
Empfangen eines ersten 3D-Bildes (121), umfassend mindestens einen Zahn (126), und ein zweites 3D-Bild (122), umfassend mindestens einen Zahn (126), wobei das zweite 3D-Bild (122) zu einem anderen Zeitpunkt aufgenommen wurde als das erste 3D-Bild (121),
Vergleichen des ersten 3D-Bildes (121) und des zweiten 3D-Bildes (122) miteinander und Bestimmen eines Differenzbetrags von Zahnbelag auf dem mindestens einen Zahn (126) basierend auf einer Abweichung zwischen dem ersten 3D-Bild (121) und dem zweiten 3D-Bild (122), und
ferner umfassend den Schritt des Übertragens eines Gitters (161), das mehrere Gittersegmente (161a - 161i) umfasst, innerhalb von mindestens dem ersten 3D-Bild (121) auf die Oberfläche des mindestens einen Zahns (126);
wobei die Gittersegmente (161a - 161i) Indexbereiche eines Dental Plaque Index darstellen, der mindestens einen Rustogi Modified Navy Plaque Index (RMNPI) und eine Turesky-Modifikation des Quigley Hein Plaque Index (TQHPI) umfasst, und wobei der Differenzbetrag an Zahnbelag auf dem mindestens einen Zahn (126) basierend auf den Indexregionen des Zahnbelagindex bestimmt wird.

2. Verfahren nach Anspruch 1, wobei mindestens eines des ersten und zweiten 3D-Bildes (121, 122) einen ersten Bildbereich umfasst, der den mindestens einen Zahn (126) zeigt, und einen zweiten Bildbereich, der das Zahnfleisch (142) zeigt, das den mindestens einen Zahn (126) umgibt, und wobei das Verfahren den Schritt des Bestimmens des ersten Bildbereichs in mindestens einem der ersten und zweiten 3D-Bilder (121, 122) durch Ausnutzen der Bildsegmentierung zum Trennen des mindestens einen Zahns (126) von dem umgebenden Zahnfleisch (142) umfasst.

3. Verfahren nach einem der Ansprüche 1 oder 2, wobei mindestens eines der ersten und zweiten 3D-Bilder (121, 122) einen ersten Bildbereich umfasst, der den mindestens einen Zahn (126) zeigt, und einen zweiten Bildbereich, der mindestens einen weiteren Zahn (127) zeigt, wobei das Verfahren ferner den Schritt des Bestimmens des ersten und zweiten Bildbereichs in mindestens einem der ersten und zweiten 3D-Bilder (121, 122) durch Ausnutzen der Bildsegmentierung unter Verwendung einer Trennfläche (51b) zwischen dem ersten und dem zweiten Bildbereich umfasst, um den mindestens einen Zahn (126) von dem mindestens einen weiteren Zahn (127) zu trennen.

4. Verfahren nach einem der Ansprüche 1 bis 3, wobei der Schritt des Bestimmens des Differenzbetrags an Zahnbelag auf dem mindestens einen Zahn (126) ein individuelles Bestimmen eines Differenzbetrags an Zahnbelag in jedem Gittersegment (161a - 161i) und ein Kombinieren der bestimmten Differenzbeträge an Zahnbelag in jedem Gittersegment (161a - 161i) umfasst, um einen gesamten Differenzbetrag an Zahnbelag des gesamten Gitters (161) zu erhalten, der auf die Oberfläche des mindestens einen Zahns (126) übertragen wird.

5. Verfahren nach einem der Ansprüche 1 bis 4, wobei der Schritt des Übertragens des Gitters (161) auf die Oberfläche des mindestens einen Zahns (126) ein Übertragen des Gitters (161) auf die Oberfläche des mindestens einen Zahns (126) durch Verankern mindestens eines Teils des Gitters (161) auf einer Segmentierungslinie umfasst, die innerhalb des mindestens ersten 3D-Bildes (121) gezeichnet ist, wobei die Segmentierungslinie zum Trennen eines ersten Bildbereichs von einem zweiten Bildbereich in dem mindestens ersten 3D-Bild (121) dient.

6. Verfahren nach einem der Ansprüche 1 bis 5, wobei das Gitter (161) darauf basiert, dass ein zuvor erzeugtes Standardgitter auf einem Standardmodellzahn erzeugt worden ist, wobei das Verfahren ferner den Schritt des Anpassens der Abmessungen des Standardgitters an die Abmessungen des mindestens einen Zahns (126) und des Anwendens des somit angepassten Standardgitters auf die Oberfläche des mindestens einen Zahns (126) umfasst.

7. Verfahren nach einem der Ansprüche 1 bis 6, ferner umfassend den Schritt des Überlagerns des ersten 3D-Bildes (121) mit dem zweiten 3D-Bild (122), wobei das erste 3D-Bild (121) den mindestens einen Zahn (126) umfasst, auf dessen Oberfläche das Gitter (161) übertragen ist, und das zweite 3D-Bild (122) den mindestens einen Zahn (126) umfasst, aber ohne ein Gitter, und des Ausrichtens des mindestens einen Zahns (126) des ersten 3D-Bildes (121) mit dem mindestens einen Zahn (126) des zweiten 3D-Bildes (122) und des Übertragens des Gitters (161) des mindestens einen Zahns (126) des ersten 3D-Bildes (121) auf die Oberfläche des mindestens einen Zahns (126) des zweiten 3D-Bildes (122).

8. Verfahren nach einem der vorstehenden Ansprüche, ferner umfassend den Schritt des Überlagerns des ersten 3D-Bildes (121) mit dem zweiten 3D-Bild (122) und Bestimmens eines Differenzbildwerts zwischen dem ersten und dem zweiten 3D-Bild (121, 122), wobei die Größe des Differenzbildwerts den Differenzbetrag an Zahnbelag auf dem mindestens einen Zahn (126) darstellt.

9. Vorrichtung (120), die zum Bestimmen von Zahnbelag konfiguriert ist, wobei die Vorrichtung (120) umfasst:
eine Schnittstelle (123), die zum Empfangen eines ersten 3D-Bildes (121), umfassend mindestens einen Zahn (126), und eines zweiten 3D-Bildes (122), umfassend mindestens einen Zahn (126), konfiguriert ist, wobei das zweite 3D-Bild (122) zu einem anderen Zeitpunkt als das erste 3D-Bild (121) aufgenommen worden ist,
eine Analyseeinheit (125), die dazu konfiguriert ist, das erste 3D-Bild (121) und das zweite 3D-Bild (122) miteinander zu vergleichen und einen Differenzbetrag (124) an Zahnbelag auf dem mindestens einen Zahn (126) basierend auf einer Abweichung zwischen dem ersten 3D-Bild (121) und dem zweiten 3D-Bild (122) zu bestimmen, und
eine Bildberechnungseinheit, die dazu konfiguriert ist, innerhalb mindestens des ersten 3D-Bildes (121) ein Gitter (161), umfassend mehrere Gittersegmente (161a bis 161i), auf die Oberfläche des mindestens einen Zahns (126) zu übertragen, wobei die Gittersegmente (161a bis 161i) Indexbereiche eines Zahnbelagindex darstellen, der mindestens einen Rustogi Modified Navy Plaque Index (RMNPI) und eine Turesky-Modifikation des Quigley Hein Plaque Index (TQHPI) umfasst, und wobei der Differenzbetrag an Zahnbelag auf dem mindestens einen Zahn (126) basierend auf den Indexbereichen des Zahnbelagindex bestimmt wird.

10. Vorrichtung (120) nach Anspruch 9, wobei die Bildberechnungseinheit dazu konfiguriert ist, das erste 3D-Bild (121) mit dem zweiten 3D-Bild (122) zum Bestimmen des Differenzbildwerts zu überlagern, der einen Differenzbetrag an Zahnbelag in jedem Gittersegment (161a bis 161i) individuell darstellt, und die Differenzbildwerte jedes Gittersegments (161a bis 161i) zu kombinieren, um einen Gesamtdifferenzbildwert zu erhalten, der einen Gesamtdifferenzbetrag an Zahnbelag des mindestens einen Zahns (126) darstellt, wobei die Größe des Gesamtdifferenzbildwerts den Gesamtdifferenzbetrag an Zahnbelag auf dem mindestens einen Zahn (126) darstellt.

## Revendications

1. Procédé mis en œuvre par ordinateur de détermination de plaque dentaire, le procédé comprenant les étapes consistant à :
recevoir une première image 3D (121) comprenant au moins une dent (126) et une deuxième image 3D (122) comprenant l'au moins une dent (126), dans lequel la deuxième image 3D (122) a été capturée à un point dans le temps différent de la première image 3D (121),
comparer la première image 3D (121) et la deuxième image 3D (122) l'une à l'autre et déterminer, sur la base d'une déviation entre la première image 3D (121) et la deuxième image 3D (122), une quantité différentielle de plaque dentaire sur l'au moins une dent (126), et
comprenant en outre l'étape consistant à transférer, au sein d'au moins la première image 3D (121), une grille (161) comprenant de multiples segments de grille (161a - 161 i) sur la surface de l'au moins une dent (126) ;
dans lequel les segments de grille (161a - 161i) représentent des régions d'indice d'un indice de plaque dentaire comprenant au moins l'un parmi un indice de plaque de Navy modifié par Rustogi (RMNPI) et une modification de Turesky de l'indice de plaque de Quigley Hein (TQHPI), et dans lequel la quantité différentielle de plaque dentaire sur l'au moins une dent (126) est déterminée sur la base des régions d'indice de l'indice de plaque dentaire.

2. Procédé selon la revendication 1, dans lequel au moins l'une des première et deuxième images 3D (121, 122) comprend une première région d'image montrant l'au moins une dent (126) et une deuxième région d'image montrant la gencive (142) entourant l'au moins une dent (126), et dans lequel le procédé comprend l'étape consistant à déterminer la première région d'image dans au moins l'une des première et deuxième images 3D (121, 122) en exploitant une segmentation d'image pour séparer l'au moins une dent (126) de la gencive environnante (142).

3. Procédé selon l'une des revendications 1 ou 2, dans lequel au moins l'une des première et deuxième images 3D (121, 122) comprend une première région d'image montrant l'au moins une dent (126) et une deuxième région d'image montrant au moins une autre dent (127), dans lequel le procédé comprend en outre l'étape consistant à déterminer les première et deuxième régions d'image dans au moins l'une des première et deuxième images 3D (121, 122) en exploitant une segmentation d'image en utilisant une surface de séparation (51b) entre les première et deuxième régions d'image pour séparer l'au moins une dent (126) de l'au moins une autre dent (127).

4. Procédé selon l'une des revendications 1 à 3, dans lequel l'étape consistant à déterminer la quantité différentielle de plaque dentaire sur l'au moins une dent (126) comprend la détermination d'une quantité différentielle de plaque dentaire dans chaque segment de grille (161a - 161i) individuellement, et la combinaison des quantités différentielles de plaque dentaire déterminées dans chaque segment de grille (161a - 161i) pour obtenir une quantité différentielle totale de plaque dentaire de la grille entière (161) transférée sur la surface de l'au moins une dent (126).

5. Procédé selon l'une des revendications 1 à 4, dans lequel l'étape consistant à transférer la grille (161) sur la surface de l'au moins une dent (126) comprend le transfert de la grille (161) sur la surface de l'au moins une dent (126) par ancrage d'au moins une partie de la grille (161) sur une ligne de segmentation tracée à l'intérieur de l'au moins première image 3D (121), la ligne de segmentation permettant de séparer une première région d'image d'une deuxième région d'image dans l'au moins première image 3D (121).

6. Procédé selon l'une des revendications 1 à 5, dans lequel la grille (161) est basée sur une grille normalisée précédemment générée ayant été générée sur une dent modèle normalisée, dans lequel le procédé comprend en outre l'étape d'ajustement des dimensions de la grille normalisée aux dimensions de l'au moins une dent (126) et d'application de la grille normalisée ainsi ajustée sur la surface de l'au moins une dent (126).

7. Procédé selon l'une des revendications 1 à 6, comprenant en outre l'étape consistant à superposer la première image 3D (121) avec la deuxième image 3D (122), dans lequel la première image 3D (121) comprend l'au moins une dent (126) ayant la grille (161) transférée sur sa surface et la deuxième image 3D (122) comprend l'au moins une dent (126) mais sans grille, et à aligner l'au moins une dent (126) de la première image 3D (121) avec l'au moins une dent (126) de la deuxième image 3D (122) et à transférer la grille (161) de l'au moins une dent (126) de la première image 3D (121) sur la surface de l'au moins une dent (126) de la deuxième image 3D (122).

8. Procédé selon l'une des revendications précédentes, comprenant en outre l'étape consistant à superposer la première image 3D (121) avec la deuxième image 3D (122) et à déterminer une valeur d'image différentielle entre les première et deuxième images 3D (121, 122), dans lequel l'ordre de grandeur de la valeur d'image différentielle représente la quantité différentielle de plaque dentaire sur l'au moins une dent (126).

9. Dispositif (120) configuré pour déterminer la plaque dentaire, le dispositif (120) comprenant :
une interface (123) configurée pour recevoir une première image 3D (121) comprenant au moins une dent (126) et une deuxième image 3D (122) comprenant l'au moins une dent (126), dans lequel la deuxième image 3D (122) a été capturée à un point dans le temps différent de la première image 3D (121),
une unité d'analyse (125) qui est configurée pour comparer la première image 3D (121) et la deuxième image 3D (122) l'une à l'autre et pour déterminer, sur la base d'une déviation entre la première image 3D (121) et la deuxième image 3D (122), une quantité différentielle (124) de plaque dentaire sur l'au moins une dent (126), et
une unité de calcul d'image étant configurée pour transférer, à l'intérieur d'au moins la première image 3D (121), une grille (161) comprenant de multiples segments de grille (161a à 161i) sur la surface de l'au moins une dent (126), dans lequel les segments de grille (161a à 161i) représentent des régions d'indice d'un indice de plaque dentaire comprenant au moins l'un parmi un indice de plaque de Navy modifié par Rustogi (RMNPI) et une modification de Turesky de l'indice de plaque de Quigley Hein (TQHPI), et dans lequel la quantité différentielle de plaque dentaire sur l'au moins une dent (126) est déterminée sur la base des régions d'indice de l'indice de plaque dentaire.

10. Dispositif (120) selon la revendication 9, dans lequel l'unité de calcul d'image est configurée pour superposer la première image 3D (121) avec la deuxième image 3D (122) pour déterminer une valeur d'image différentielle représentant une quantité différentielle de plaque dentaire dans chaque segment de grille (161a à 161i) individuellement, et pour combiner les valeurs d'image différentielles de chaque segment de grille (161a à 161i) pour obtenir une valeur d'image différentielle totale représentant une quantité différentielle totale de plaque dentaire de l'au moins une dent (126), dans lequel l'ordre de grandeur de la valeur d'image différentielle totale représente la quantité différentielle totale de plaque dentaire sur l'au moins une dent (126).
